# FASCICULE DE BREVET EUROPEEN

(11) **EP 3 522 957 B1**
(45) Date de publication et mention de la délivrance du brevet: **22.07.2020**
(21) Numéro de dépôt: 17793987.3
(22) Date de dépôt: 02.10.2017
(51) Int. Cl.: A61M 5/32, A61M 5/50, A61M 5/315

(54) **DISPOSITIF D'INJECTION MANUELLE**
MANUELLE INJEKTIONSVORRICHTUNG
MANUAL INJECTION DEVICE

(30) Priorité: 04.10.2016 FR 1659543
(43) Date de publication de la demande: 14.08.2019
(73) Titulaire: Aptar France SAS, 27110 Le Neubourg (FR)
(72) Inventeur: SAUSSAYE, Anthony, 27400 Louviers (FR); JAOUEN, Quentin, 76220 Gournay en Bray (FR)
(74) Mandataire: CAPRI
(86) Numéro de dépôt international: PCT/FR2017/052691
(87) Numéro de publication internationale: WO 2018/065708

(56) Documents cités:
- FR-A1- 3 013 601

## Description

La présente invention concerne un dispositif d'injection manuelle.

Les dispositifs d'injection manuelle ont principalement pour but de réaliser la pénétration de l'aiguille dans le corps du patient ainsi que la protection de l'aiguille de la seringue avant, pendant et après l'injection. Par contre, l'injection dans le corps du patient du produit contenu dans la seringue est réalisée manuellement par l'utilisateur. Les dispositifs d'injection manuelle sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces dispositifs d'injection manuelle étant à usage unique, le coût de fabrication et d'assemblage est également un facteur dont il faut tenir compte.

Il existe de nombreux dispositifs d'injection manuelle sur le marché, qui présentent toutefois tous un certain nombre d'inconvénients.

Ainsi, il ne faut pas que l'utilisation du dispositif d'injection manuelle soit trop difficile, ce qui empêcherait des personnes faibles de l'utiliser. Il faut aussi éviter que l'injection ne commence avant que l'aiguille ait pénétré dans le corps du patient. De plus, afin d'éviter tout risque de blessures avant et après l'utilisation du dispositif, le dispositif d'injection manuelle doit comprendre un dispositif de sécurité aiguille qui évite que l'aiguille ne soit apparente avant et après l'utilisation du dispositif. Ce dispositif de sécurité doit évidemment également être fiable et ne pas se libérer trop facilement. Il doit aussi être fonctionnel même si l'utilisateur actionne mal le dispositif d'injection manuelle, par exemple s'il le retire trop tôt de son corps, avant la fin de l'injection.

Les documents WO2015075399, WO2014150201, WO2011047298, WO2006129196 et WO2006111862 décrivent des dispositifs de l'état de la technique.

La présente invention a pour but de fournir un dispositif d'injection manuelle qui ne reproduit pas les inconvénients susmentionnés, et qui permet de répondre aux différentes exigences et contraintes importantes pour une utilisation sûre et fiable du dispositif d'injection manuelle.

La présente invention a notamment but de fournir un dispositif d'injection manuelle qui évite le risque que l'injection du produit ne débute avant que l'aiguille ait complètement pénétrée le site d'injection.

La présente invention a aussi pour but de fournir un dispositif d'injection manuelle qui soit fiable d'utilisation, qui soit sûr et qui empêche tout risque de blessure, et qui soit simple et peu coûteux à fabriquer et à assembler.

La présente invention a donc pour objet un dispositif d'injection manuelle comportant :
- un corps inférieur recevant un réservoir, ledit réservoir étant axialement fixe par rapport audit corps inférieur et contenant du produit fluide à injecter, ledit réservoir comportant un piston et une aiguille,
- un corps supérieur axialement déplaçable par rapport audit corps inférieur lors de l'actionnement, ledit corps supérieur comportant une tige de piston coopérant avec ledit piston lors de l'injection pour le déplacer dans le réservoir,
- un manchon actionneur comportant une extrémité de contact destinée à venir en contact avec le corps de l'utilisateur, ledit manchon actionneur étant déplaçable par rapport audit corps inférieur entre des positions projetées, dans lesquelles ledit manchon actionneur fait au moins partiellement saillie hors dudit corps inférieur, et une position d'actionnement, dans laquelle ledit manchon actionneur est axialement déplacé vers l'intérieur dudit corps inférieur, ledit manchon actionneur étant dans une première position projetée avant actionnement du dispositif d'injection manuelle et dans une seconde position projetée après actionnement du dispositif d'injection manuelle,
ledit manchon actionneur définissant avec ledit corps inférieur, ou avec tout élément solidaire dudit corps inférieur, tel qu'un support de réservoir, une serrure de piquage, et ladite tige de piston définit avec ledit réservoir, ou avec tout élément solidaire dudit réservoir, tel qu'un support de réservoir ou tel que ledit corps inférieur, une serrure d'injection, la force nécessaire pour actionner ladite serrure de piquage étant inférieure à la force nécessaire pour actionner ladite serrure d'injection, de sorte que ladite serrure de piquage est actionnée avant ladite serrure d'injection.

Avantageusement, l'un parmi ledit manchon actionneur et ledit corps inférieur, ou tout élément solidaire dudit corps inférieur, comporte une patte flexible adaptée à se déformer latéralement par rapport audit manchon actionneur et/ou par rapport audit corps inférieur lorsque ledit manchon actionneur est déplacé de sa première position projetée vers sa position d'actionnement puis de sa position d'actionnement en retour vers sa seconde position projetée, l'autre parmi ledit manchon actionneur et ledit corps, ou tout élément solidaire dudit corps inférieur, comportant une zone initiale qui coopère avec ladite patte flexible dans ladite première position projetée, une zone intermédiaire qui coopère avec ladite patte flexible dans ladite position d'actionnement, et une zone de réception finale qui coopère avec ladite patte flexible dans ladite seconde position projetée, ladite zone de réception finale étant décalée au moins latéralement de ladite zone initiale.

Avantageusement, une paroi axiale déformable est adaptée à se déformer élastiquement pour laisser passer ladite patte flexible de ladite zone initiale vers ladite zone intermédiaire, ladite paroi axiale déformable, dans sa position non déformée, étant ensuite adaptée à guider ladite patte flexible de ladite zone intermédiaire vers ladite zone de réception finale.

Avantageusement, ladite zone de réception finale est reliée à ladite zone intermédiaire par une rainure finale, un épaulement axial étant prévu entre ladite zone de réception finale et ladite rainure finale, ladite patte flexible étant adaptée à coulisser dans ladite rainure finale lorsque ledit manchon actionneur revient de sa position d'actionnement vers sa seconde position projetée, ladite patte flexible venant s'encliqueter sous ledit épaulement axial lorsque ledit manchon actionneur atteint sa seconde position projetée, après utilisation, verrouillant ainsi ledit manchon actionneur par rapport audit corps inférieur.

Selon un premier mode de réalisation avantageux, ladite tige de piston comporte un évidement central définissant deux branches flexibles qui s'écartent radialement pour former un épaulement radialement externe de chaque côté de ladite tige de piston.

Avantageusement, lesdits épaulements radialement externes coopèrent avec une bride axiale solidaire d'un élément fixé audit corps inférieur.

Avantageusement, lesdits épaulements radialement externes coopèrent avec une collerette radiale dudit réservoir.

Avantageusement, chaque épaulement radialement externe définit un profil indenté, lesdits profils indentés recevant une projection radiale d'une bride axiale solidaire d'un élément fixé audit corps inférieur.

Selon un second mode de réalisation avantageux, ladite tige de piston, avant actionnement, est reliée par des ponts sécables à un élément fixe par rapport audit réservoir.

Avantageusement, avant actionnement, ladite tige de piston est reliée à un disque fixé audit corps inférieur.

En variante, avant actionnement, ladite tige de piston est reliée à une bague fixée, notamment sertie, sur une collerette radiale dudit réservoir.

Selon un mode de réalisation avantageux, ladite tige de piston, avant actionnement, est reliée à une bague fixée, notamment sertie, sur une collerette radiale dudit réservoir, ladite bague comportant une projection radialement interne coopérant, avant actionnement, avec une gorge de ladite tige de piston.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
la figure 1 est une vue schématique en section transversale d'un dispositif d'injection manuelle selon un premier mode de réalisation avantageux, avant utilisation,
la figure 2 est une vue similaire à celle de la figure 1, après retrait du capuchon de protection de l'aiguille, en position de repos avant piquage,
la figure 3 est une vue similaire à celle de la figure 2, en fin de piquage, avant déclenchement de la serrure d'injection,
la figure 4 est une vue similaire à celle de la figure 3, après déclenchement de la serrure d'injection,
la figure 5 est une vue similaire à celle de la figure 4, en début d'injection,
la figure 6 est une vue similaire à celle de la figure 5, en cours d'injection,
la figure 7 est une vue similaire à celle de la figure 6, en fin d'injection, avant déclenchement du dispositif de sécurité post-injection,
la figure 8 est une vue similaire à celle de la figure 7, en fin d'injection, après déclenchement du dispositif de sécurité post-injection,
la figure 9 est une vue similaire à celle de la figure 8, en fin d'utilisation, après actionnement du dispositif de sécurité post-injection,
les figures 10 et 11 sont des vues de détail d'une première variante de réalisation de la serrure d'injection,
les figures 12 à 14 sont des vues de détail d'une seconde variante de réalisation de la serrure d'injection,
les figures 15 à 18 sont des vues de détail d'une variante de réalisation du système de blocage du piston après injection,
la figure 19 est une vue schématique en section transversale d'un dispositif d'injection manuelle selon un second mode de réalisation avantageux, avant utilisation,
la figure 20 est une vue similaire à celle de la figure 19, en début de piquage,
la figure 21 est une vue similaire à celle de la figure 20, en fin de piquage, avant déclenchement de la serrure d'injection,
la figure 22 est une vue similaire à celle de la figure 21, en début d'injection,
la figure 23 est une vue similaire à celle de la figure 22, en fin d'injection,
la figure 24 est une vue similaire à celle de la figure 23, en fin d'utilisation, après actionnement du dispositif de sécurité post-injection,
les figures 25 à 29 sont des vues de détail de plusieurs variantes de réalisation de la serrure d'injection,
Les figures 30 à 35 sont des vues schématiques partielles illustrant en détail la serrure de piquage lors des différentes séquences d'utilisation du dispositif d'injection manuelle de la figure 1, et
Les figures 36 à 38 sont des vues schématiques partielles illustrant des variantes de réalisation de la serrure de piquage.

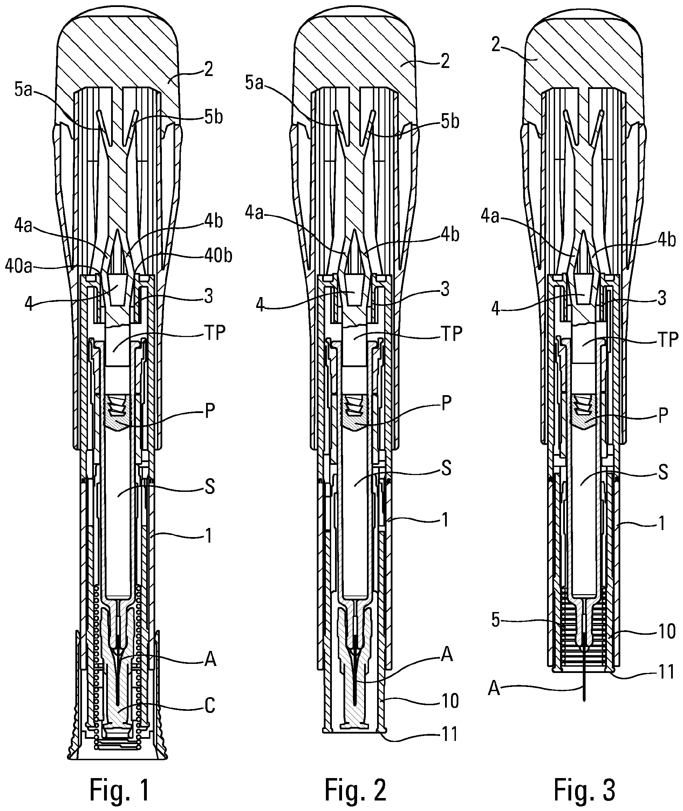

Le dispositif d'injection manuelle représenté sur les figures comporte un corps inférieur 1 et un corps supérieur 2, axialement déplaçable par rapport audit corps inférieur 1 lors de l'actionnement. Il est à noter que les corps inférieur 1 et supérieur 2 peuvent être réalisés de manière monobloc ou en plusieurs parties assemblées. Dans ledit corps inférieur 1 coulisse axialement un manchon actionneur 10, dont l'extrémité inférieure 11 (dans l'orientation de la figure 1) est destinée à venir en contact avec le corps du patient autour de la zone d'injection. De manière connue, ce corps inférieur 1 contient un réservoir S contenant le produit à injecter, une aiguille A fixée audit réservoir S à travers laquelle le produit est distribué et un piston P adapté à se déplacer dans ledit réservoir S pour réaliser cette injection. Le corps supérieur 2 comporte une tige de piston TP coopérant avec ledit piston P lors de l'injection pour le déplacer dans le réservoir S. L'aiguille A peut être protégée avant utilisation par un capuchon de protection C, représenté sur la figure 1. Le réservoir S peut typiquement être une seringue pré-remplie classique, pourvue d'une collerette radiale 9. Ledit réservoir S est fixe par rapport audit corps inférieur 1 et ladite tige de piston TP est fixe par rapport audit corps supérieur 2. Ainsi, lorsque le corps supérieur 2 coulisse axialement par rapport au corps inférieur 1, la tige de piston TP coulisse axialement par rapport au réservoir S.

Avant actionnement, le manchon actionneur 10 est dans une première position projetée dans laquelle il entoure l'aiguille A, formant ainsi une sécurité pré-injection. Lors de l'actionnement, le manchon actionneur 10 coulisse à l'intérieur du corps inférieur 1 vers une position d'actionnement, pour exposer l'aiguille A et permettre le piquage et l'injection du produit fluide. Après l'injection, le manchon actionneur 10 revient dans une seconde position projetée, dans laquelle il est à nouveau disposé autour de l'aiguille A, pour éviter tout risque de blessure avec ladite aiguille A, formant ainsi un dispositif de sécurité post-injection. Le manchon actionneur 10 est avantageusement sollicité vers ses positions projetées par un ressort 5, qui peut être de type quelconque.

Le manchon actionneur 10 définit avec le corps inférieur 1 (ou avec tout élément solidaire dudit corps inférieur 1, tel que par exemple le support de réservoir) une serrure de piquage et la tige de piston TP définit avec le réservoir S (ou avec tout élément solidaire dudit réservoir S, tel que par exemple le support de réservoir ou le corps inférieur) une serrure d'injection.

Selon l'invention, la serrure de piquage est actionnée avant la serrure d'injection, c'est à dire que la force axiale nécessaire pour déclencher la serrure de piquage est inférieure à la force axiale nécessaire pour déclencher la serrure d'injection. Ainsi, lorsque l'utilisateur appuie le dispositif contre le site d'injection et appuie axialement sur le corps supérieur 2 pour le faire coulisser axialement par rapport au corps inférieur 1, c'est d'abord le manchon actionneur 10 qui se déplace axialement à l'intérieur du corps inférieur 1 pour réaliser le piquage. La tige de piston TP ne se déplace axialement à l'intérieur du corps inférieur 1 qu'après que ledit manchon actionneur 10 a atteint sa position d'actionnement.

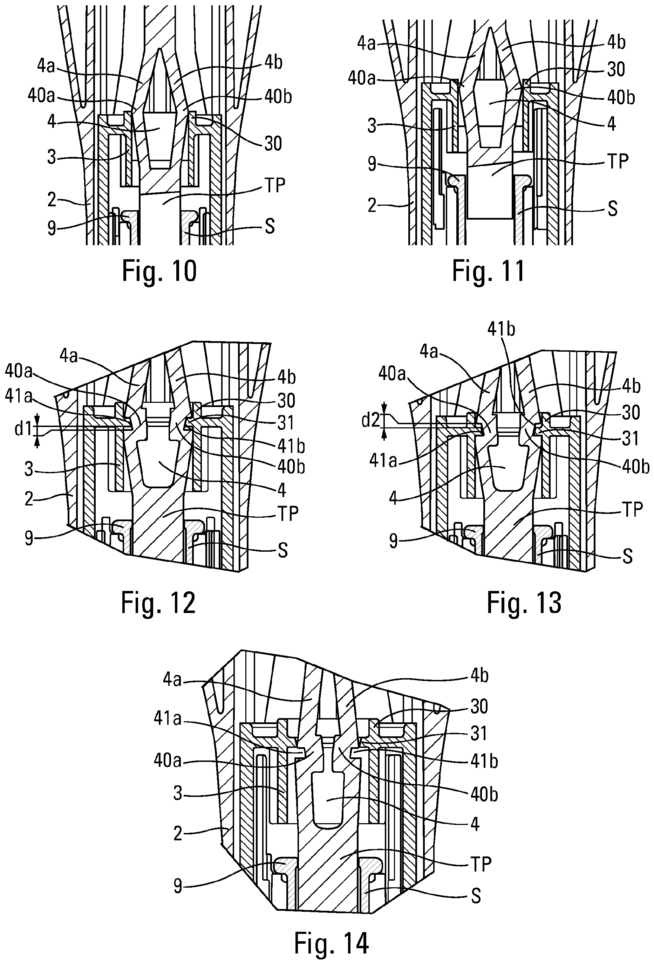

Les figures 1 à 18 illustrent un premier mode de réalisation avantageux. Les figures 1 à 9 montrent différentes positions dans la séquence d'actionnement du dispositif d'injection manuelle, et les figures 10 à 18 montrent des variantes de réalisation de la serrure d'injection ainsi que le système de blocage du piston après injection.

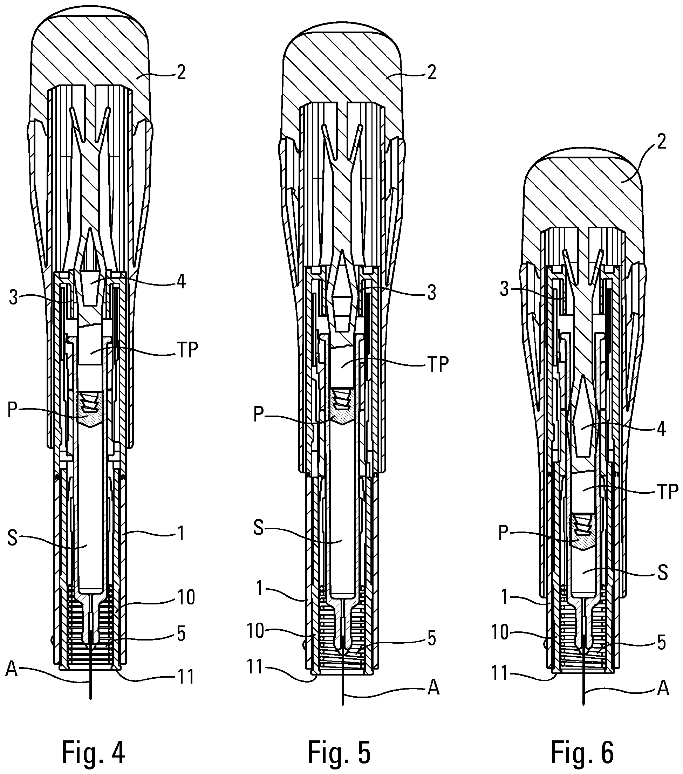

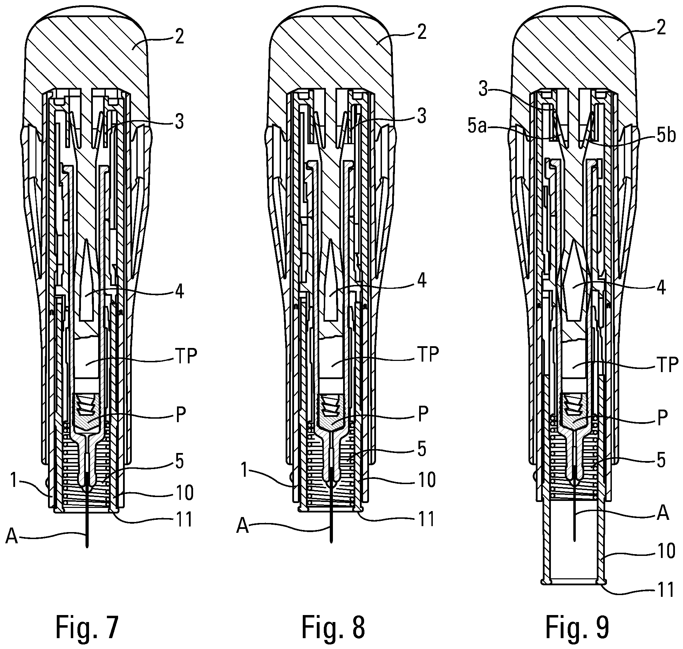

En référence aux figures 1 à 9, on constate qu'après retrait du capuchon de protection C, l'utilisateur appuie l'extrémité inférieure 11 du manchon actionneur 10 autour du site d'injection, et exerce une pression axiale sur le corps supérieur 2. La résistance au déclenchement de la serrure d'injection, qui sera décrite ultérieurement, étant supérieure à celle de la serrure de piquage, qui sera également décrite ultérieurement, c'est d'abord le manchon actionneur 10 qui va coulisser axialement vers l'intérieur du corps inférieur 1. Ceci va découvrir l'aiguille A, qui va donc pénétrer dans le site d'injection sous l'effet de ladite pression axiale de l'utilisateur. Ce n'est que lorsque le manchon actionneur 10 atteint sa position d'actionnement, visible sur les figures 3 et 4, que la force d'actionnement ou pression axiale de l'utilisateur servira à déclencher la serrure d'injection. Après déclenchement de ladite serrure d'injection, le corps supérieur 2 et donc la tige de piston TP vont se déplacer axialement par rapport au corps inférieur 1 et donc par rapport au réservoir S, pour déplacer le piston P dans le réservoir S et ainsi injecter le produit contenu dans la réservoir, à travers l'aiguille A, dans le site d'injection. En fin d'injection, lorsque l'utilisateur retire le dispositif d'injection manuelle du site d'injection, le manchon actionneur 10 revient automatiquement, par exemple sous l'effet du ressort de rappel 5, vers sa seconde position projetée, dans laquelle il se verrouille, pour éviter tout risque de blessure avec l'aiguille A, comme visible sur la figure 9.

Les figures 10 et 11 illustrent une première variante de réalisation de la serrure d'injection. Dans cette variante, la tige de piston TP comporte un évidement central 4 définissant deux branches flexibles 4a, 4b qui s'écartent radialement pour former un épaulement radialement externe 40a, 40b de chaque côté de la tige de piston TP. Ces épaulements 40a, 40b coopèrent avec une bride axiale 30 solidaire d'un élément 3 fixé audit corps inférieur 1. Pour déclencher la serrure d'injection, il faut déformer lesdites branches 4a, 4b radialement vers l'intérieur, pour permettre auxdits épaulements 40a, 40b de passer axialement au-delà de ladite bride 30. La force axiale nécessaire pour ce faire est supérieure à celle nécessaire pour déclencher la serrure de piquage, ce qui garantit que l'injection ne commencera qu'après la fin du piquage.

Les figures 12 à 14 montrent une autre variante de réalisation de la serrure de piquage. Ici, les deux épaulements radialement externes 40a, 40b définissent chacun un profil indenté 41a, 41b, ces profils indentés recevant une projection radiale 31 de ladite bride axiale 30. Dans cette variante, il est fourni en outre une fonction anti-arrachement, de par l'encliquetage des profils 41a, 41b sur la projection 31, empêchant de retirer la tige de piston TP hors du réservoir S. De préférence, on prévoit un jeu pour cet encliquetage, illustré sur les figures 12 et 13 par les distances d1 et d2.

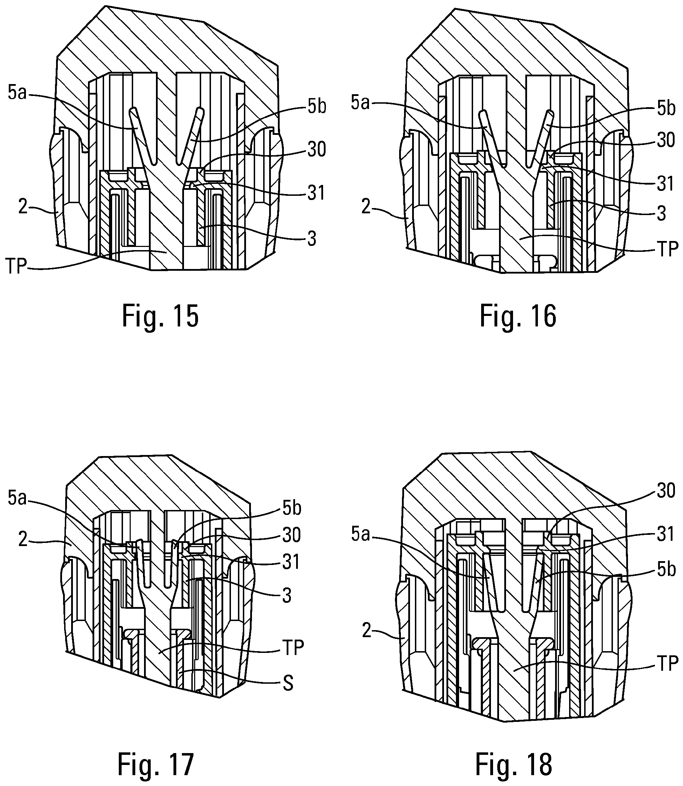

Les figures 15 à 18 illustrent une variante de réalisation d'un dispositif de blocage du piston après utilisation. Dans cet exemple, la tige de piston TP comporte dans sa partie arrière (par rapport au piston P) deux pattes flexibles 5a, 5b s'étendant axialement vers l'arrière en s'évasant radialement vers l'extérieur. En fin d'injection, ces pattes flexibles 5a, 5b se déforment radialement vers l'intérieur au passage de la projection 31 de la bride 30, pour venir s'encliqueter dessous, comme visible sur la figure 18. Ceci bloque la tige de piston TP dans le corps inférieur 1 et donc le piston P dans le réservoir S. Bien entendu, ce dispositif pourrait être adapté à une serrure de piquage selon les figures 10 et 11, qui pourrait alors comporter une projection 31 sur sa bride axiale 30.

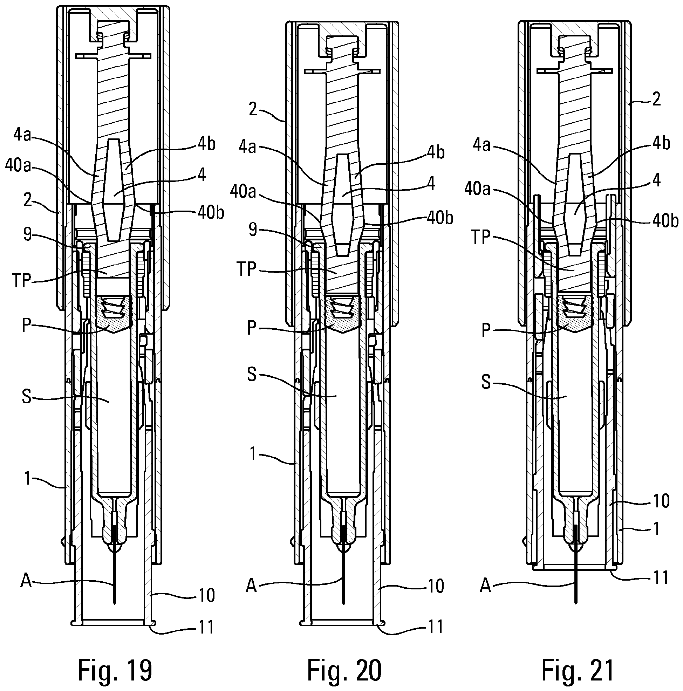

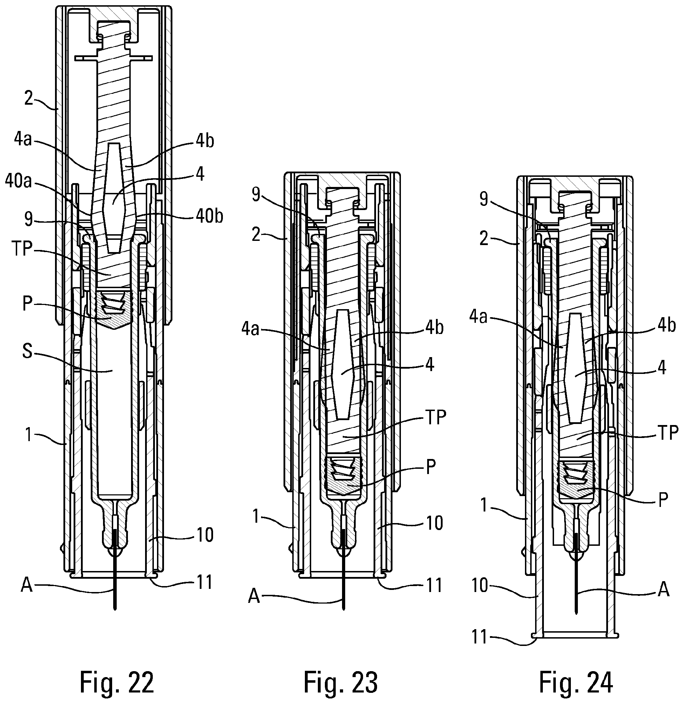

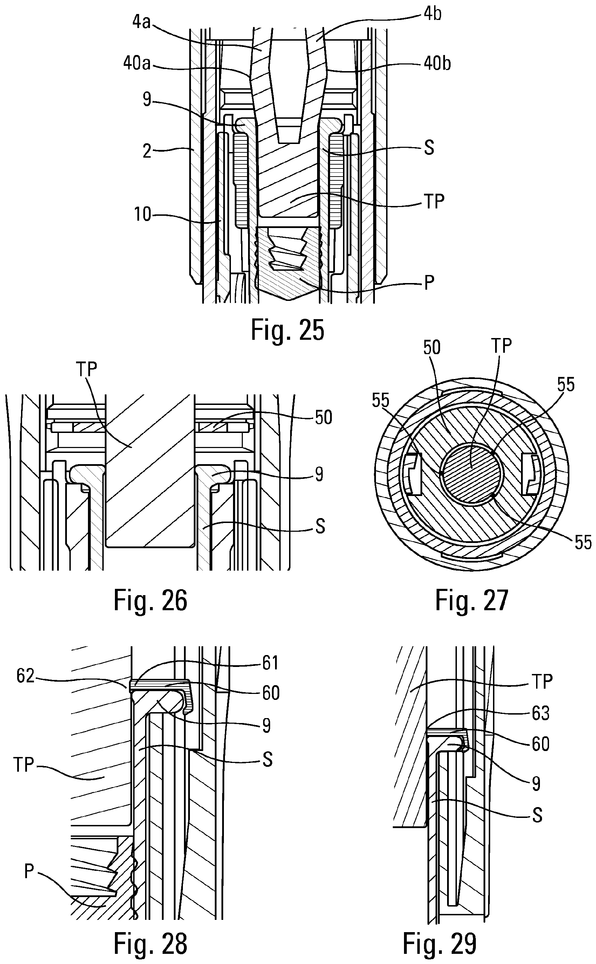

Les figures 19 à 29 illustrent un second mode de réalisation avantageux. Les figures 19 à 24 montrent différentes positions dans la séquence d'actionnement du dispositif d'injection manuelle, et les figures 25 à 29 montrent des variantes de réalisation de la serrure d'injection. La séquence d'actionnement est similaire à celle du premier mode de réalisation, avec d'abord le piquage, puis l'injection.

La structure du dispositif d'injection manuelle est simplifiée, avec la tige de piston TP encliquetée au niveau de son extrémité arrière (opposée au piston P) dans le corps supérieur 2.

La principale différence concerne la serrure d'injection, formée sur les figures 19 à 25 entre la tige de piston TP et la collerette radiale 9 de la seringue S. dans cette variante de réalisation, la tige de piston TP est réalisée comme dans le premier mode de réalisation avec un évidement central 4 définissant deux branches 4a, 4b qui s'écartent radialement pour former un épaulement 40a, 40b de chaque côté. Ces épaulements 40a, 40b coopèrent avec ladite collerette radiale 9 de la seringue S.

Sur les figures 26 et 27 est représentée une autre variante avantageuse, dans laquelle un disque 50, fixe par rapport au corps inférieur 1 et donc par rapport au réservoir S, est reliée avant actionnement à la tige de piston TP par des ponts sécables 55, typiquement trois comme visible sur la figure 27. La résistance à la rupture de ces ponts sécables 55 est supérieure à la force de déclenchement de la serrure de piquage, de sorte que le piquage a lieu avant l'injection.

La figure 28 illustre une autre variante dans laquelle une bague de sertissage 60 est fixée directement sur la collerette radiale 9 de la seringue, ladite bague 60 comportant une projection radialement interne 61 coopérant avant actionnement avec une gorge 62 de la tige de piston TP. La force nécessaire pour désengager ladite projection 61 de ladite gorge 62 est supérieure à la force de déclenchement de la serrure de piquage, de sorte que le piquage a lieu avant l'injection.

La figure 29 illustre encore une autre variante dans laquelle ladite projection radialement interne 61 de bague de sertissage 60 est fixée à ladite tige de piston TP par des ponts sécables 63. La résistance à la rupture de ces ponts sécables 63 est supérieure à la force de déclenchement de la serrure de piquage, de sorte que le piquage a lieu avant l'injection.

D'autres variantes de réalisation de la serrure de piquage sont envisageables.

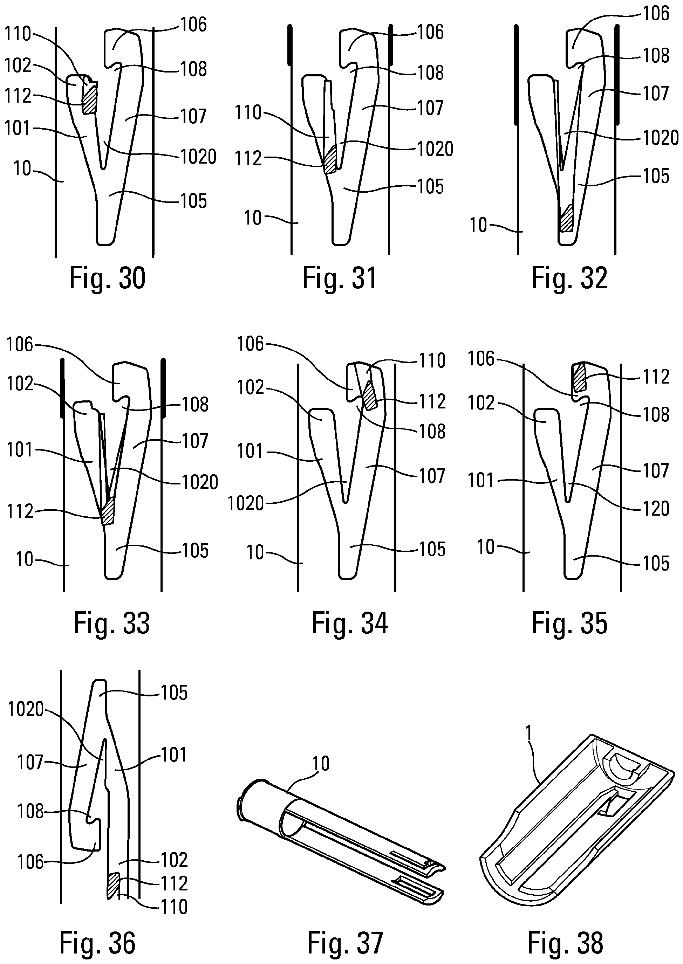

Les figures 30 à 38 illustrent des modes de réalisation avantageux de la serrure de piquage et du dispositif de sécurité pré- et post-injection. Ces dispositifs s'appliquent aux deux modes de réalisation et aux diverses variantes décrits ci-dessus.

Avantageusement, l'un parmi ledit manchon actionneur 10 et ledit corps inférieur 1 (ou un élément solidaire dudit corps inférieur 1) comporte une patte flexible 110 adaptée à se déformer latéralement par rapport audit manchon actionneur 10 et/ou par rapport audit corps inférieur 1 recevant le réservoir S lorsque ledit manchon actionneur 10 est déplacé de sa première position projetée vers sa position d'actionnement puis de sa position d'actionnement en retour vers sa seconde position projetée.

Un premier mode de réalisation avantageux d'une serrure de piquage est représenté sur les figures 30 à 35. Dans ce mode de réalisation, le manchon actionneur 10 comporte une rainure initiale 101, avantageusement inclinée, qui s'étend depuis une zone initiale 102 vers une zone intermédiaire 105. Ladite rainure initiale 101 comporte de préférence une paroi axiale 1020 élastiquement déformable. Ledit manchon actionneur 10 comporte aussi une zone de réception finale 106 décalée au moins latéralement par rapport à ladite zone initiale 102, et reliée à ladite zone intermédiaire 105 par une rainure finale 107, avantageusement inclinée. Un épaulement axial 108 est prévu entre ladite zone de réception finale 106 et ladite rainure finale 107.

Le corps inférieur 1 comporte une patte 110 flexible latéralement, c'est-à-dire qu'elle se déforme dans la direction périphérique du corps. Cette patte flexible 110 comporte avantageusement une tête 112 qui va coopérer avec les rainures et épaulements du manchon actionneur 10, comme cela sera décrit ci-après.

Les figures 30 à 35 illustrent plus particulièrement le fonctionnement de la serrure de piquage formée entre le manchon actionneur 10 et le corps inférieur 1.

La figure 30 représente la position de départ, c'est-à-dire au moment où l'utilisateur va commencer à utiliser le dispositif d'injection manuelle. On voit sur cette figure 30 que la tête 112 de la patte flexible 110 est disposée dans ladite zone initiale 102. Lorsque le manchon actionneur 10 coulisse vers l'intérieur du corps inférieur 1, ladite tête 112 de la patte flexible 110 va coulisser à l'intérieur de ladite rainure initiale 101. Lorsque la tête 112 atteint la position de la figure 31, dans laquelle elle coopère d'un côté avec la rainure initiale 101 et de l'autre côté avec la paroi axiale déformable 1020, elle va déformer latéralement ladite paroi axiale déformable 1020, pour pouvoir continuer sa course d'actionnement. La déformation de la paroi axiale déformable 1020 permet à la tête 112 de la patte flexible 110 de passer dans la zone intermédiaire 105. Eventuellement, au moment de la déformation maximale de ladite paroi axiale déformable 1020, celle-ci peut générer un son, tel qu'un clic, pour signaler à l'utilisateur que l'injection va débuter.

Dans la position représentée sur la figure 32, le manchon actionneur 10 a atteint sa position d'actionnement dans laquelle l'aiguille A a pénétré dans la zone d'injection du patient jusqu'à la position d'injection et dans laquelle l'injection peut commencer. Dans cette position d'actionnement du manchon actionneur 10, la tête 112 de la patte flexible 110 est dans la zone intermédiaire 105. Dans cette position, la paroi axiale déformable 1020 est revenue élastiquement vers sa position non déformée, de sorte que ladite zone intermédiaire 105 est reliée à ladite zone de réception finale 106 par ladite rainure finale inclinée 107.

Lorsque ledit manchon actionneur 10 revient de sa position d'actionnement vers sa seconde position projetée sous l'effet du ressort 5, ladite tête 112 de la patte flexible 110 coulisse dans ladite rainure finale inclinée 107. La figure 33 illustre schématiquement que la patte flexible 110, en particulier la tête 112, ne peut plus revenir dans ladite rainure initiale 101 en raison de la paroi axiale déformable 1020 qui empêche le passage de ladite tête 112. Avantageusement, ladite tête 112 comporte une paroi frontale au moins partiellement inclinée qui va coopérer avec l'extrémité inclinée de ladite paroi axiale déformable 1020 pour guider ladite tête dans la rainure finale inclinée 107. Ainsi, la paroi axiale déformable 1020 fait partie intégrante de la came, formée par la rainure finale 107, qui va guider la patte flexible 110 vers la zone de réception finale 106.

Lorsque ledit manchon actionneur 10 atteint sa seconde position projetée, après utilisation, ladite tête 112 vient s'encliqueter dans ladite zone de réception finale 106 sous ledit épaulement axial 108, verrouillant ainsi ledit manchon actionneur 10 par rapport audit corps inférieur 1. A partir de cette position verrouillée, ledit manchon actionneur 10 ne peut plus être déplacé en direction de sa position d'actionnement, de par la butée formée entre la tête 112 de la patte flexible 110 et l'épaulement axial 108. La déformation latérale de la patte flexible 110 lors de l'actionnement, notamment dans la rainure finale inclinée 107 comme illustré sur la figure 34, provoque un encliquetage élastique automatique de la tête 112 sous l'épaulement axial 108 dès que la tête 112 arrive dans ladite zone de réception finale 106, comme illustré sur la figure 35. Le dispositif de sécurité post-injection est alors en position finale verrouillée. Ainsi, l'aiguille A est totalement protégée après utilisation et l'utilisateur ne peut plus utiliser le dispositif d'injection manuelle et/ou se blesser avec l'aiguille. Eventuellement, cet encliquetage dans la zone de réception finale 106 peut générer un son, tel qu'un clic, pour informer l'utilisateur que la position finale verrouillée est atteinte.

La serrure de piquage du manchon actionneur décrite ci-dessus est particulièrement efficace et fiable, tout en étant robuste et facile, et donc peu coûteuse, à mouler et à assembler. En particulier, elle ne comporte que deux pièces, le manchon actionneur 10 et le corps inférieur 1.

Bien entendu, les formes des rainures, leurs dimensions et leurs inclinaisons peuvent être modifiées en fonction des besoins et des caractéristiques souhaitées pour le dispositif de sécurité aiguille. En particulier, la rainure initiale peut être axiale ou inclinée. Elle peut mener directement à la zone intermédiaire, sans qu'il n'y ait de seconde et troisième rainures. Avec une rainure initiale inclinée, la rainure finale pourrait être axiale ou aussi inclinée. De plus, la serrure de piquage ne comporte pas nécessairement la paroi axiale déformable 1020 décrite ci-dessus

D'autres variantes sont envisageables.

En particulier, la patte flexible 110 pourrait être formée sur un élément solidaire dudit corps inférieur 1 et non directement dans ledit corps inférieur 1. En particulier, cette patte flexible 110 pourrait être formée sur le support du réservoir S, qui maintient fixement ledit réservoir dans ledit corps inférieur 1.

Il est à noter que les moyens décrits ci-dessus pourraient être réalisés de manière inversée, c'est-à-dire que le corps 1 (ou un élément solidaire dudit corps 1, tel que le support de réservoir) pourrait comporter les diverses rainures et épaulement et le manchon actionneur 10 pourrait comporter la patte flexible 110. Dans ce cas, les formes et orientations desdites rainures seraient bien entendu adaptées en conséquence. Les figures 36 à 38 illustrent schématiquement une telle variante.

Bien que la présente invention ait été décrite en référence à des modes de réalisation avantageux, il est entendu que l'homme du métier peut y apporter diverses modifications sans sortir du cadre de la présente invention tel que défini par les revendications annexées.

## Revendications

1. Dispositif d'injection manuelle comportant :
- un corps inférieur (1) recevant un réservoir (S), ledit réservoir étant axialement fixe par rapport audit corps inférieur (1) et contenant du produit fluide à injecter, ledit réservoir (S) comportant un piston (P) et une aiguille (A),
- un corps supérieur (2) axialement déplaçable par rapport audit corps inférieur (1) lors de l'actionnement, ledit corps supérieur (2) comportant une tige de piston (TP) coopérant avec ledit piston (P) lors de l'injection pour le déplacer dans le réservoir (S),
- un manchon actionneur (10) comportant une extrémité de contact (11) destinée à venir en contact avec le corps de l'utilisateur, ledit manchon actionneur (10) étant déplaçable par rapport audit corps inférieur (1) entre des positions projetées, dans lesquelles ledit manchon actionneur (10) fait au moins partiellement saillie hors dudit corps inférieur (1), et une position d'actionnement, dans laquelle ledit manchon actionneur (10) est axialement déplacé vers l'intérieur dudit corps inférieur (1), ledit manchon actionneur (10) étant dans une première position projetée avant actionnement du dispositif d'injection manuelle et dans une seconde position projetée après actionnement du dispositif d'injection manuelle,
ledit manchon actionneur (10) définissant avec ledit corps inférieur (1), ou avec tout élément solidaire dudit corps inférieur (1), tel qu'un support de réservoir, une serrure de piquage, **caractérisé en ce que** ladite tige de piston (TP) définit avec ledit réservoir (S), ou avec tout élément solidaire dudit réservoir (S), tel qu'un support de réservoir ou tel que ledit corps inférieur (1), une serrure d'injection, la force nécessaire pour actionner ladite serrure de piquage étant inférieure à la force nécessaire pour actionner ladite serrure d'injection, de sorte que ladite serrure de piquage est actionnée avant ladite serrure d'injection.

2. Dispositif d'injection manuelle selon la revendication 1, dans lequel l'un parmi ledit manchon actionneur (10) et ledit corps inférieur (1), ou tout élément solidaire dudit corps inférieur (1), comporte une patte flexible (110) adaptée à se déformer latéralement par rapport audit manchon actionneur (10) et/ou par rapport audit corps inférieur (1) lorsque ledit manchon actionneur (10) est déplacé de sa première position projetée vers sa position d'actionnement puis de sa position d'actionnement en retour vers sa seconde position projetée, l'autre parmi ledit manchon actionneur (10) et ledit corps (1), ou tout élément solidaire dudit corps inférieur (1), comportant une zone initiale (102) qui coopère avec ladite patte flexible (110) dans ladite première position projetée, une zone intermédiaire (105) qui coopère avec ladite patte flexible (110) dans ladite position d'actionnement, et une zone de réception finale (106) qui coopère avec ladite patte flexible (110) dans ladite seconde position projetée, ladite zone de réception finale (106) étant décalée au moins latéralement de ladite zone initiale (102).

3. Dispositif d'injection manuelle selon la revendication 2, dans lequel une paroi axiale déformable (1020) est adaptée à se déformer élastiquement pour laisser passer ladite patte flexible (110) de ladite zone initiale (102) vers ladite zone intermédiaire (105), ladite paroi axiale déformable (1020), dans sa position non déformée, étant ensuite adaptée à guider ladite patte flexible de ladite zone intermédiaire (105) vers ladite zone de réception finale (106).

4. Dispositif d'injection manuelle selon la revendication 2 ou 3, dans lequel ladite zone de réception finale (106) est reliée à ladite zone intermédiaire (105) par une rainure finale (107), un épaulement axial (108) étant prévu entre ladite zone de réception finale (106) et ladite rainure finale (107), ladite patte flexible (110) étant adaptée à coulisser dans ladite rainure finale (107) lorsque ledit manchon actionneur (10) revient de sa position d'actionnement vers sa seconde position projetée, ladite patte flexible (110) venant s'encliqueter sous ledit épaulement axial (108) lorsque ledit manchon actionneur (10) atteint sa seconde position projetée, après utilisation, verrouillant ainsi ledit manchon actionneur (10) par rapport audit corps inférieur (1).

5. Dispositif d'injection manuelle selon l'une quelconque des revendications précédentes, dans lequel ladite tige de piston (TP) comporte un évidement central (4) définissant deux branches flexibles (4a, 4b) qui s'écartent radialement pour former un épaulement radialement externe (40a, 40b) de chaque côté de ladite tige de piston (TP).

6. Dispositif d'injection manuelle selon la revendication 5, dans lequel lesdits épaulements radialement externes (40a, 40b) coopèrent avec une bride axiale (30) solidaire d'un élément (3) fixé audit corps inférieur (1).

7. Dispositif d'injection manuelle selon la revendication 5, dans lequel lesdits épaulements radialement externes (40a, 40b) coopèrent avec une collerette radiale (9) dudit réservoir (S).

8. Dispositif d'injection manuelle selon la revendication 5, dans lequel chaque épaulement radialement externe (40a, 40b) définit un profil indenté (41a, 41b), lesdits profils indentés (41a, 41b) recevant une projection radiale (31) d'une bride axiale (30) solidaire d'un élément (3) fixé audit corps inférieur (1).

9. Dispositif d'injection manuelle selon l'une quelconque des revendications 1 à 4, dans lequel ladite tige de piston (TP), avant actionnement, est reliée par des ponts sécables (55, 63) à un élément (50, 60) fixe par rapport audit réservoir (S).

10. Dispositif d'injection manuelle selon la revendication 9, dans lequel, avant actionnement, ladite tige de piston (TP) est reliée à un disque (50) fixé audit corps inférieur (1).

11. Dispositif d'injection manuelle selon la revendication 9, dans lequel, avant actionnement, ladite tige de piston (TP) est reliée à une bague (60) fixée, notamment sertie, sur une collerette radiale (9) dudit réservoir (S).

12. Dispositif d'injection manuelle selon l'une quelconque des revendications 1 à 4, dans lequel ladite tige de piston (TP), avant actionnement, est reliée à une bague (60) fixée, notamment sertie, sur une collerette radiale (9) dudit réservoir (S), ladite bague (60) comportant une projection radialement interne (61) coopérant, avant actionnement, avec une gorge (62) de ladite tige de piston (TP).

## Patentansprüche

1. Manuelle Injektionsvorrichtung, aufweisend:
- einen Unterkörper (1), der einen Behälter (S) aufnimmt, wobei der Behälter bezogen auf den Unterkörper (1) axial fest ist und zu injizierendes Fluidprodukt enthält, wobei der Behälter (S) einen Kolben (P) und eine Nadel (A) aufweist,
- einen Oberkörper (2), der bezogen auf den Unterkörper (1) während der Betätigung axial bewegbar ist, wobei der Oberkörper (2) eine Kolbenstange (TP) aufweist, die mit dem Kolben (P) während der Injektion zusammenwirkt, um ihn in dem Behälter (S) zu bewegen,
- eine Betätigungshülse (10) aufweisend ein Kontaktende (11), das dazu bestimmt ist, mit dem Körper des Benutzers in Kontakt zu treten, wobei die Betätigungshülse (10) bezogen auf den Unterkörper (1) zwischen vorstehenden Positionen, in welchen die Betätigungshülse (10) zumindest teilweise aus dem Unterkörper (1) hervorsteht, und einer Betätigungsposition, in welcher die Betätigungshülse (10) axial zum Inneren des Unterkörpers (1) bewegt ist, bewegbar ist, wobei sich die Betätigungshülse (10) vor Betätigung der manuellen Injektionsvorrichtung in einer ersten vorstehenden Position und nach Betätigung der manuellen Injektionsvorrichtung in einer zweiten vorstehenden Position befindet,
wobei die Betätigungshülse (10) mit dem Unterkörper (1) oder mit einem beliebigen Element, das fest mit dem Unterkörper (1) verbunden ist, wie beispielsweise einem Behälterhalter, ein Einstechschloss definiert, **dadurch gekennzeichnet, dass**
die Kolbenstange (TP) mit dem Behälter (S) oder mit einem beliebigen Element, das fest mit dem Behälter (S) verbunden ist, wie beispielsweise einem Behälterträger oder dem Unterkörper (1) ein Injektionsschloss definiert, wobei die Kraft, die notwendig ist, um das Einstechschloss zu betätigen, geringer als die Kraft ist, die notwendig ist, um das Injektionsschloss zu betätigen, so dass das Einstechschloss vor dem Injektionsschloss betätigt wird.

2. Manuelle Injektionsvorrichtung nach Anspruch 1, wobei eins von der Betätigungshülse (10) und dem Unterkörper (1) oder jedes beliebige Element, das fest mit dem Unterkörper (1) verbunden ist, einen flexiblen Ansatz (110) aufweist, der geeignet ist, sich seitlich bezogen auf die Betätigungshülse (10) und/oder bezogen auf den Unterkörper (1) zu verformen, wenn die Betätigungshülse (10) aus ihrer ersten vorstehenden Position zu ihrer Betätigungsposition, dann aus ihrer Betätigungsposition zurück zu ihrer zweiten vorstehenden Position bewegt wird, das andere von der Betätigungshülse (10) und dem Körper (1) oder jedes beliebige Element, das fest mit dem Unterkörper (1) verbunden ist, aufweisend einen Initialbereich (102), der mit dem flexiblen Ansatz (110) in der ersten vorstehenden Position zusammenwirkt, einen Zwischenbereich (105), der mit dem flexiblen Ansatz (110) in der Betätigungsposition zusammenwirkt, und einen Endaufnahmebereich (106), der mit dem flexiblen Ansatz (110) in der zweiten vorstehenden Position zusammenwirkt, wobei der Endaufnahmebereich (106) zumindest seitlich von dem Initialbereich (102) versetzt ist.

3. Manuelle Injektionsvorrichtung nach Anspruch 2, wobei eine verformbare Axialwand (1020) geeignet ist, sich elastisch zu verformen, um den flexiblen Ansatz (110) aus dem Initialbereich (102) zu dem Zwischenbereich (105) passieren zu lassen, wobei die verformbare Axialwand (1020) dann in ihrer nicht verformten Position geeignet ist, den flexiblen Ansatz aus dem Zwischenbereich (105) zu dem Endaufnahmebereich (106) zu führen.

4. Manuelle Injektionsvorrichtung nach Anspruch 2 oder 3, wobei der Endaufnahmebereich (106) durch eine Endnut (107) mit dem Zwischenbereich (105) verbunden ist, wobei ein Axialabsatz (108) zwischen dem Endaufnahmebereich (106) und der Endnut (107) vorgesehen ist, wobei der flexible Ansatz (110) geeignet ist, in die Endnut (107) zu gleiten, wenn die Betätigungshülse (10) aus ihrer Betätigungsposition zu ihrer zweiten vorstehenden Position zurückkehrt, wobei der flexible Ansatz (110) unter dem Axialabsatz (108) einrastet, wenn die Betätigungshülse (10) nach Gebrauch ihre zweite vorstehende Position erreicht, wobei so die Betätigungshülse (10) bezogen auf den Unterkörper (1) verriegelt wird.

5. Manuelle Injektionsvorrichtung nach einem der vorhergehenden Ansprüche, wobei die Kolbenstange (TP) eine Mittelaussparung (4) aufweist, die zwei flexible Zweige (4a, 4b) definiert, die radial auseinander gehen, um einen radial äußeren Absatz (40a, 40b) auf jeder Seite der Kolbenstange (TP) zu bilden.

6. Manuelle Injektionsvorrichtung nach Anspruch 5, wobei die radial äußeren Absätze (40a, 40b) mit einem Axialflansch (30) zusammenwirken, der fest mit einem Element (3) verbunden ist, das an dem Unterkörper (1) befestigt ist.

7. Manuelle Injektionsvorrichtung nach Anspruch 5, wobei die radial äußeren Absätze (40a, 40b) mit einem Radialkragen (9) des Behälters (S) zusammenwirken.

8. Manuelle Injektionsvorrichtung nach Anspruch 5, wobei jeder radial äußere Absatz (40a, 40b) ein gezahntes Profil (41a, 41b) definiert, wobei die gezahnten Profile (41a, 41b) einen Radialvorsprung (31) eines Axialflanschs (30) aufnehmen, der fest mit einem Element (3) verbunden ist, das an dem Unterkörper (1) befestigt ist.

9. Manuelle Injektionsvorrichtung nach einem der Ansprüche 1 bis 4, wobei die Kolbenstange (TP) vor Betätigung durch abtrennbare Brücken (55, 63) mit einem Element (50, 60) verbunden ist, das bezogen auf den Behälter (S) fest ist.

10. Manuelle Injektionsvorrichtung nach Anspruch 9, wobei vor Betätigung die Kolbenstange (TP) mit einer Scheibe (50) verbunden ist, die an dem Unterkörper (1) befestigt ist.

11. Manuelle Injektionsvorrichtung nach Anspruch 9, wobei vor Betätigung die Kolbenstange (TP) mit einem Ring (60) verbunden ist, der an einem Radialkragen (9) des Behälters (S) befestigt, insbesondere gecrimpt ist.

12. Manuelle Injektionsvorrichtung nach einem der Ansprüche 1 bis 4, wobei die Kolbenstange (TP) vor Betätigung mit einem Ring (60) verbunden ist, der an einem Radialkragen (9) des Behälters (S) befestigt, insbesondere gecrimpt ist, der Ring (60) aufweisend einen radial inneren Vorsprung (61), der vor Betätigung mit einer Rille (62) der Kolbenstange (TP) zusammenwirkt.

## Claims

1. A manual injection device comprising:
▪ a lower body (1) that receives a reservoir (S), said reservoir being stationary axially relative to said lower body (1) and containing fluid to be injected, said reservoir (S) including a piston (P) and a needle (A),
▪ an upper body (2) that is movable axially relative to said lower body (1) during actuation, said upper body (2) including a piston rod (TP) that co-operates with said piston (P) during injection so as to move it in the reservoir S,
▪ an actuator sleeve (10) that includes a contact end (11) for coming into contact with the user's body, said actuator sleeve (10) being movable relative to said lower body (1) between projecting positions, in which said actuator sleeve (10) projects at least in part out from said lower body (1), and an actuated position in which said actuator sleeve (10) is moved axially into said lower body (1), said actuator sleeve (10) being in a first projecting position before the manual injection device has been actuated and in a second projecting position after the manual injection device has been actuated,
said actuator sleeve (10) defining with said lower body (1), or with any element that is secured to said lower body (1), such as a reservoir support, a pricking lock, **characterized in that** said piston rod (TP) defines with said reservoir (S), or with any element that is secured to said reservoir (S), such as a reservoir support or such as said lower body (1), an injection lock, the force necessary for actuating said pricking lock being less than the force necessary for actuating said injection lock, such that said pricking lock is actuated before said injection lock.

2. A manual injection device according to claim 1, wherein one of said actuator sleeve (10) and said lower body (1), or any element that is secured to said lower body (1), includes a flexible tab (110) that is adapted to deform laterally relative to said actuator sleeve (10) and/or relative to said lower body (1) when said actuator sleeve (10) is moved from its first projecting position to its actuated position and then from its actuated position while returning to its second projecting position, the other of said actuator sleeve (10) and said body (1), or any element that is secured to said lower body (1), including an initial zone (102) that co-operates with said flexible tab (110) in said first projecting position, an intermediate zone (105) that co-operates with said flexible tab (110) in said actuated position, and a final reception zone (106) that co-operates with said flexible tab (110) in said second projecting position, said final reception zone (106) being offset at least laterally from said initial zone (102).

3. A manual injection device according to claim 2, wherein a deformable axial wall (1020) is adapted to deform resiliently so as to allow said flexible tab (110) to pass from said initial zone (102) to said intermediate zone (105), said deformable axial wall (1020), in its non-deformed position, then being adapted to guide said flexible tab from said intermediate zone (105) to said final reception zone (106).

4. A manual injection device according to claim 2 or claim 3, wherein said final reception zone (106) is connected to said intermediate zone (105) via a final groove (107), an axial shoulder (108) being provided between said final reception zone (106) and said final groove (107), said flexible tab (110) being adapted to slide in said final groove (107) when said actuator sleeve (10) returns from its actuated position to its second projecting position, said flexible tab (110) becoming snap-fastened below said axial shoulder (108) when said actuator sleeve (10) reaches its second projecting position, after use, thereby locking said actuator sleeve (10) relative to said lower body (1).

5. A manual injection device according to any preceding claim, wherein said piston rod (TP) includes a central recess (4) that defines two flexible branches (4a, 4b) that slope radially away from each other so as to form a radially-outer shoulder (40a, 40b) on each side of said piston rod (TP).

6. A manual injection device according to claim 5, wherein said radially-outer shoulders (40a, 40b) co-operate with an axial flange (30) that is secured to an element (3) that is fastened to said lower body (1).

7. A manual injection device according to claim 5, wherein said radially-outer shoulders (40a, 40b) co-operate with a radial collar (9) of said reservoir (S).

8. A manual injection device according to claim 5, wherein each radially-outer shoulder (40a, 40b) defines an indented profile (41a, 41b), said indented profiles (41a, 41b) receiving a radial projection (31) of an axial flange (30) that is secured to an element (3) that is fastened to said lower body (1).

9. A manual injection device according to any one of claims 1 to 4, wherein, said piston rod (TP), before actuation, is connected via breakable bridges (55, 63) to an element (50, 60) that is stationary relative to said reservoir (S).

10. A manual injection device according to claim 9, wherein, before actuation, said piston rod (TP), is connected to a disk (50) that is fastened to said lower body (1).

11. A manual injection device according to claim 9, wherein, before actuation, said piston rod (TP) is connected to a ring (60) that is fastened, in particular crimped, on a radial collar (9) of said reservoir (S).

12. A manual injection device according to any one of claims 1 to 4, wherein said piston rod (TP), before actuation, is connected to a ring (60) fastened, in particular crimped, on a radial collar (9) of said reservoir (S), said ring (60) including a radially-inner projection (61) co-operates, before actuation, with a groove (62) of said piston rod (TP).
